# EUROPEAN PATENT APPLICATION

(11) **EP 0 711 536 A1**
(43) Date of publication of application: **15.05.1996**
(21) Application number: 95306084.5
(22) Date of filing: 31.08.1995
(51) Int. Cl.: A61F 5/44

(54) **Urine collection device**

(30) Priority: 14.11.1994 JP 279270/94
(71) Applicant: Cawood, Charles David, Houston, TX 77030 (US)
(72) Inventor: Cawood, Charles David, Houston, TX 77030 (US)
(74) Representative: Kensett, John Hinton

(57) **Abstract**

A flat urinary drainage bag (11) that can be worn by a patient over the abdomen with the bag suspended from a waist-encircling belt is disclosed. A urethral catheter (13) has a proximal end communicating with the upper portion of the bag just below waist level and a distal end retained within the patient's bladder. The device includes a valve-equipped drain tube (34) and an anti-reflux flap valve (30) within the bag at its inlet for preventing reverse flow of fluid from the bag back into the catheter. A multiplicity of baffles in the form of an arrangement of spaced heat seals (33) between the thermoplastic front and rear walls of the bag maintain the bag in relatively flat condition and impede the surge of its liquid contents from one side to the other as a patient moves about.

## Description

Conventional urinary drainage bags are commonly strapped to a patient's leg above the knee, as disclosed in U.S. patent 3,897,785, so that urine will flow into the bag under the influence of gravity. For an ambulatory patient, such an arrangement is often inconvenient and uncomfortable because, as such a bag becomes filled with urine, there is a tendency for it to slide downwardly along the leg, unless additional means are provided to restrain such sliding movement. Also, such leg bags may be conspicuous through clothing as the bags become filled and may be awkward to drain.

It is now found that gravity flow is not essential for the purposes of filling a urine collection bag and that intrinsic bladder detrusor muscle tone and intraperitoneal pressures exerted upon the bladder of a catheterized ambulatory patient will cause urine to flow from the bladder to a level as high as 10 centimeters or more above the distal tip of the catheter. A highly effective urinary drainage system may therefore be provided for an ambulatory patient in which the collection bag is carried by a waistband or belt and is worn over the patient's abdomen instead of along the inside of the leg.

Such a bag may be comfortably worn by a patient and, because of its flatness, is generally inconspicuous beneath clothing. Such flatness is assured by a multiplicity of spaced-apart heat seals joining the front and rear walls of the bag, such heat seals also being shaped, positioned and arranged to serve also as baffles for impeding and deflecting the surging flow of the liquid contents of such a bag as the aumbulatory patient moves about. The heat seals, which are preferably vertically elongated and staggered, therefore prevent the sloshing of liquid from one side of the bag to the other and contribute in maintaining quiteness as well as inconspicuousness of the bag in use.

Both the catheter and the bag's drain tube may be relatively short. By positioning the drain tube at a central point along the bag's lower edge, a male wearer may conveniently drain the contents of the bag by simply unzipping his pants, extracting the bag's drainage tube, and opening the drain valve. Both male and female patients have the convenience of being able to empty the urinary collection bags by assuming positions customarily taken during voiding by those who have no urinary afflictions or disabilities, unlike wearers of leg bags who must, in order to drain such bags, adjust their clothing to gain access to the drain tubes located at, or even below, knee level.

Briefly, the collection device takes the form of a flat bag having front and rear walls of flexible thermoplastic material joined to each other along top, bottom, and side edges. Means are provided along the top edge for engaging the belt to support the bag from a patient's waist. A valve-equipped drain tube is located along the bottom edge and communicates with the interior of the bag, and an inlet tube is joined to one of the walls above the drain tube and is adapted to be connected to a urethral catheter. A one-way inlet valve communicates with the inlet tube for preventing the flow of urine in a reverse direction. A plurality of heat seals attach the front and rear walls together at a multiplicity of spaced attachment zones positioned inwardly from the side edges of the bag, such heat seals maintaining the walls in close proximity, so that the bag has a relatively flat profile even when full, and impeding or deflecting the flow of the liquid contents to prevent sloshing actions and noises that might create distraction and embarrassment.

The inlet tube is connected to the proximal end of a conventional urethral catheter equipped with an inflatable balloon at its distal end for retention in the urethra. When the bag is worn, the distal tip of the catheter will normally be about 5 to 10 centimeters below the inlet of the bag; however, intrinsic detrusor muscle tone of the bladder and intraperitoneal pressures associated with common body action such as walking, bending, and breathing result in fluid flow from the bladder into the bag with the anti-refluxing valve preventing reverse flow through the catheter.

Embodiments of the present invention will now be described by way of example only, with reference to the accompanying drawings, in which
Figure 1 is a front elevational view of a urine collection device.
Figure 2 is a vertical sectional view taken along line 2-2 of Figure 1.
Figure 3 is a front elevational view of the bag, as shown partly in vertical section, illustrating the flow-directing baffles therein.
Figure 4 is a front elevational view of the device as it would be worn by a catherized male patient.

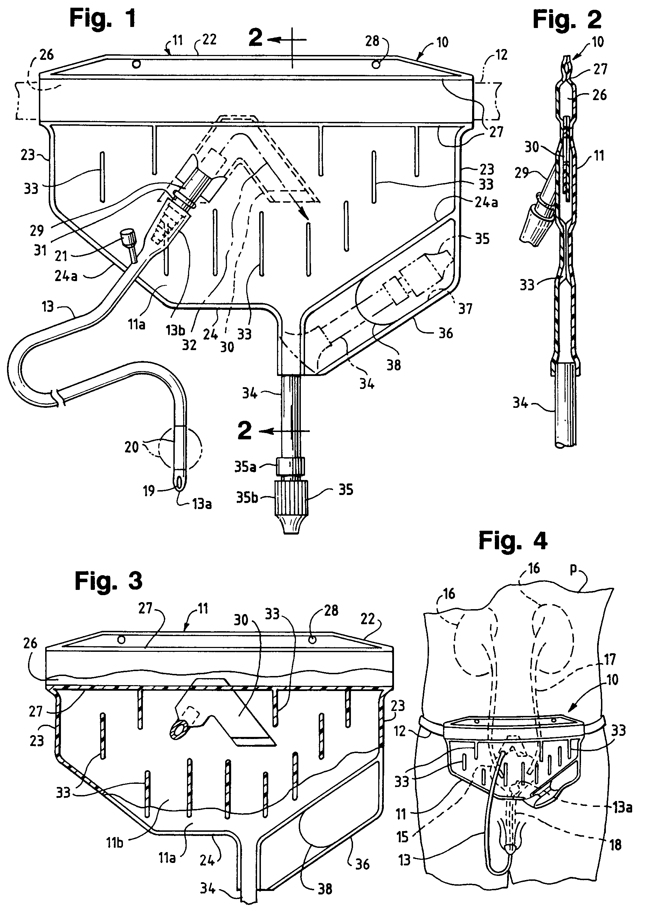

Referring to the drawings, the numeral 10 generally designates a urine collection device comprising an abdominal bag 11, a belt 12 for supporting the bag about a patient's waist, and a catheter 13 for conveying urine from the bladder to the collection bag. Figure 4 illustrates the device as it could be worn by a male patient with catheter 13 extending through the urethra and the distal end 13a of the catheter being disposed within bladder 15. Urine drains into the bladder from kidneys 16 and ureters 17. The urethral sphincter located at 18 would normally control flow from the bladder 15; however, the sphincter is rendered inoperative or ineffective by catheter 13, with the result that urine is free to flow from the bladder into the catheter. Catheter 13 is a conventional retention catheter, commonly called a Foley catheter, having an inlet 19 at its distal end 13a. An inflatable balloon 20 is disposed near the tip of the catheter and may be inflated into the expanded condition depicted in broken lines in Figures 1 and 4 to provide retention means for retaining the distal tip of the catheter within the neck of the bladder. A stem 21 providing a suitable self-sealing inflation port is located at the proximal end 13b of the catheter. Inflation and deflation of the balloon is achieved by inserting a needle of a syringe into the inflation port, all as well known in the art.

Bag 11 is flat when empty and is dimensioned to extend over a patient's abdomen or belly as shown in Figure 4. Specifically, the bag has front and rear panels or walls 11a and 11b, respectively joined together along their top side and bottom edges 22-24. Top edge 22 is generally straight and extends horizontally when the bag is worn. Immediately below the top edge is a horizontally-elongated tubular channel 26 defined by parallel heat seal lines 27, the channel serving as a horizontally-elongated belt loop for receiving belt 12 and thereby supporting or suspending the bag from a patient's waist. Alternatively, belt 12 may be formed integrally with the bag, or may take the form of a pair of straps permanently secured to the bag and projecting laterally therefrom. The construction shown is preferred because bag 11 is a disposable item formed of a suitable thermoplastic material such as, for example, a polyolefin film (e.g., polyethylene) laminated with an appropriate barrier material (e.g., polyvinylidene chloride), whereas the belt would ordinarily be formed of cloth and would be reusable. A belt formed of cotton or any other soft breathable fabric is believed particularly effective, such belt being equipped with a conventional clasp or buckle (not shown) allowing adjustment of the size of the belt so that it extends snugly but comfortably about a wearer's waist.

If desired, the upper edge portion of the bag 11 may be provided with openings 28 that may be used to secure the upper edge of the bag to the patient's undergarments by means of safety pins or other suitable fasteners.

The dimensions of the bag may be varied depending on the size of the patient. In general, the bag should have a width within the range of about 20 to 40 centimeters (preferably about 30 centimeters) and a height of about 10 to 20 centimeters (preferably about 15 centimeters). In any event, the bag should be dimensioned to extend generally over the wearer's abdomen, from his (her) waist down to the pelvic region, as depicted in Figure 4. When the bag is so worn, it is positioned at approximately the same height as the wearer's bladder 15.

An inlet tube 29 formed of polyvinyl chloride or other suitable thermoplastic material is heat sealed to the upper front wall 11a of the bag and communicates in the interior of the bag to a suitable one-way valve 30. As shown in the drawings, the exterior portion of the inlet tube is operatively connected to the proximal end 13b of catheter 13. The connection might be a permanent one, although a separable connection is preferred. To facilitate coupling and uncoupling of the catheter and tube and at the same time achieve a secure connection that will not become accidentally disrupted, it has been found desirable to seal a connecting sleeve or nipple 31 to the outer end of the inlet tube, the nipple being stepped as shown (Figure 1) and being formed of a relatively rigid material such as, for example, polystyrene.

The one-way valve 30 may be formed of a pair of inverted V-shaped flexible thermoplastic strips 30a and 30b heat sealed along their inner and outer edges to define a passage communicating at one end with inlet tube 29 and open at its other end only when fluid pressure within the passage forces the strips 30a and 30b apart at that other end. The anti-refluxing flap valve 30 is centrally positioned within the upper portion of the bag about equal distances from side edges 23 and may be advantageously secured in place by the lower heat seal line 27 that also defines the upper limits of the bag's interior and the lower limits of the belt-receiving channel 26. It will be understood that the same heat seal area that secures inlet tube 29 to the bag, with the inlet tube communicating with the passage of flap valve 30, also seals off the leg of the valve passage immediately adjacent tube 29, with the result that fluid may flow through the valve passage only in the direction indicated generally by arrow 32 in Figure 1.

As shown most clearly in Figure 3, the side walls 11a and 11b of the bag are heat sealed to each other at a multiplicity of attachment zones 33 spaced throughout the fluid-receiving body portion of the bag. The heat seals 33 are vertically elongated and, in the illustration given, are generally parallel with side edges 23. The length of each heat seal 33 is substantially less than the maximum vertical dimension of the fluid-receiving chamber and are laterally (horizontally) spaced apart in vertically-staggered or offset relation. It is important that a plurality of such heat seals 33 be provided, although the particular number (10) shown in the drawings is not critical and a somewhat greater or smaller number may be provided as desired. Such heat seals are positioned and arranged so that fluid within the bag cannot flow from one side edge 23 to the other without encountering and being deflected by one or more seals. The heat seals 33 therefore function as flow-deflecting baffles, and the provision of multiple baffles disperses flow and retards the rate of flow to eliminate or greatly reduce the sloshing of fluid, and the sounds associated with it, as a patient moves about. In addition, the multiple heat seals 33 limit the extent of outward bulging of the side walls of the bag as it is filled and promote more uniform distribution of liquid throughout the width and height of the bag, thereby insuring that the bag maintains a relatively flat and inconspicuous profile even when it is filled.

A short drain tube 34 formed of polyvinyl chloride or other flexible thermoplastic material is heat sealed to the lower edges of the bag and communicates with the bag as most clearly shown in Figures 1 and 2. At its free end, the drain tube is equipped with a suitable valve 35. The particular valve depicted in the drawings is composed of two elements 35a and 35b that are threadedly connected to each other; opening and closing of the valve is achieved simply by rotating element 35b one way or the other with respect to element 35a. Since such a valve is entirely conventional and well known for use in collection appliances, a more detailed discussion of its structure and operation is believed unnecessary.

Portions 24a of the bag's lower edge 24 slope downwardly and inwardly to direct fluid towards the centrally-disposed drain tube 34, as depicted in Figure 1. In the embodiment illustrated, one lower side portion of the bag extends downwardly and is heat sealed along lower edge 36 to define a pocket 37. Slit 38 forms the entrance to that pocket. The drain tube 34 and valve 35 may be inserted into the pocket, as shown in Figure 4 and in broken lines in Figure 1, to restrain movement of the drain tube during normal wearing of the bag. When draining of the contents of the bag is desired, the tube is simply removed from the pocket and valve 35 is manipulated into open condition. For reasons already given, such a procedure is far more convenient for the patient than those involved in the draining of a conventional leg bag.

While the urinary collection appliance is shown as it would be worn by a male patient, it is to be understood that the appliance is equally useful for female patients. In both cases, the intrinsic bladder detrusor muscle tone and the intraperitoneal pressure created during normal body movements or actions, such as breathing, walking, and bending, provide sufficient pressure to direct urine upwardly through the short length of catheter 13 and into the inlet tube 29 and one-way valve 30 in the upper central portion of the collection bag. Therefore, despite the fact that the urine collection bag is carried by a belt or waistband over the patient's abdomen, urine from the bladder is readily directed into the bag. Reverse flow, especially as might otherwise occur if the patient were sitting or reclining, is prevented by the anti-refluxing flap valve 30.

While in the foregoing, an embodiment of the invention has been described in detail for purposes of illustration, it will be understood by those skilled in the art that many of these details may be varied without departing from the scope of the invention.

## Claims

1. A urine collection device comprising a flat bag (11) adapted to be worn by a patient across the abdomen; said bag having front and rear walls (11a,11b) of flexible thermoplastic material joined to each other along top, bottom, and side edges to define a urine-receiving chamber; means along said top edge for engaging a belt (12) to support said bag from a patient's waist; a valve-equipped drain tube (34) located along said bottom edge and communicating with the interior of said bag; an inlet tube (29) joined to one of said walls above said drain tube and adapted to be connected to a urethral catheter; a one-way inlet valve (30) communicating with said inlet tube for preventing flow in a reverse direction therethrough; said front and rear walls of said bag being joined to each other at a multiplicity of spaced attachment zones in the form of heat seals (33) located throughout said urine-receiving chamber for maintaining said walls of said chamber in proximity and for deflecting the flow of liquid contents of said bag as a patient moves about.

2. The device of Claim 1 in which said heat seals are elongated in directions extending generally vertically when said bag is worn and said patient is standing erect.

3. The device of Claim 2 in which said heat seals are spaced horizontally apart and vertically staggered.

4. The device of Claim 2 in which each of said vertically-elongated heat seals has a length substantially less than the maximum vertical dimension of said chamber.

5. The device of Claim 1 in which said means comprises at least one transversely-elongated channel (26) for receiving a belt adapted to be fitted about a patient's waist.

6. The device of Claim 1 in which said device includes a urethral catheter (13) having proximal and distal ends; the proximal end of said catheter being secured to said inlet tube and the distal end of said catheter including inflatable and deflatable retention means (20).

7. The device of Claim 1 in which said drain tube is centrally disposed along said bottom edge and said bottom edge includes lateral portions (24a) sloping downwardly and inwardly from said side edges towards said drain tube.

8. The device of Claim 7 in which said drain tube is flexible; said bag being provided with a pocket (37) having an opening (38) in said front wall for receiving the free end portion of said drain tube when said drain tube is not in use.
